# EUROPEAN PATENT SPECIFICATION

(11) **EP 2 099 489 B1**
(45) Date of publication and mention of the grant of the patent: **21.05.2014**
(21) Application number: 07761925.2
(22) Date of filing: 04.05.2007
(51) Int. Cl.: A61K 39/395, A61P 35/00

(54) **COMPOSITIONS AND METHODS FOR TREATING A NEOPLASM**
ZUSAMMENSETZUNGEN UND VERFAHREN ZUR BEHANDLUNG EINER NEOPLASIE
COMPOSITIONS ET PROCEDES DE TRAITEMENT D'UN NEOPLASME

(30) Priority: 11.12.2006 US 874460 P
(43) Date of publication of application: 16.09.2009
(73) Proprietor: Genentech, Inc., South San Francisco CA 94080-4990 (US)
(72) Inventor: MASS, Robert, D., Mill Valley, California 94941 (US); PLOWMAN, Gregory, D., San Carlos, California 94070 (US)
(74) Representative: Denison, Christopher Marcus
(86) International application number: PCT/US2007/068300
(87) International publication number: WO 2008/073509

(56) References cited:
- DATABASE BIOSIS [Online] BIOSCIENCES INFORMATION SERVICE, PHILADELPHIA, PA, US; November 2006 (2006-11), SIEGEL DAVID S ET AL: "Phase II trial of SCIO-469 as monotherapy (M) or in combination with bortezornib (MB) in relapsed refractory multiple myeloma (MM)." XP002489623 Database accession no. PREV200700260327 & BLOOD, vol. 108, no. 11, Part 1, November 2006 (2006-11), page 1022A, 48TH ANNUAL MEETING OF THE AMERICAN-SOCIETY-OF-HEMATOLOGY; ORLANDO, FL, USA; DECEMBER 09 -12, 2006 ISSN: 0006-4971
- DATABASE BIOSIS [Online] BIOSCIENCES INFORMATION SERVICE, PHILADELPHIA, PA, US; 16 November 2003 (2003-11-16), RICHARDSON P G ET AL: "A multi-center, randomized, phase 2 study to evaluate the efficacy and safety of 2 CDC-5013 dose regimens when used alone or in combination with dexamethasone (dex) for the treatment of relapsed or refractory multiple myeloma (MM)." XP002489624 Database accession no. PREV200400133332 & BLOOD, vol. 102, no. 11, 16 November 2003 (2003-11-16), page 235a, 45TH ANNUAL MEETING OF THE AMERICAN SOCIETY OF HEMATOLOGY; SAN DIEGO, CA, USA; DECEMBER 06-09, 2003 ISSN: 0006-4971
- MOTZER R J ET AL: "Targeted therapy for metastatic renal cell carcinoma" JOURNAL OF CLINICAL ONCOLOGY 20061210 US, vol. 24, no. 35, 10 December 2006 (2006-12-10), pages 5601-5608, XP009103715 ISSN: 0732-183X
- PEINERT ET AL.: "Salvage therapy with bevacizumab, capecitabin, and mitomycin C (BECAM) for patients with metastatic colorectal or gastric cancer refractory to 5-fluorouracil, oxaliplatin, irinotecan, and cetuximab" EUROPEAN JOURNAL OF CANCER. SUPPLEMENT, PERGAMON, OXFORD, GB, vol. 3, no. 2, 1 October 2005 (2005-10-01), page 226, XP005132907 ISSN: 1359-6349
- BIDUS ET AL: "Sustained response to bevacizumab in refractory well-differentiated ovarian neoplasms" GYNECOLOGIC ONCOLOGY, ACADEMIC PRESS, LONDON, GB, vol. 102, no. 1, 1 July 2006 (2006-07-01), pages 5-7, XP005485641 ISSN: 0090-8258
- COHN ET AL: "Bevacizumab and weekly taxane chemotherapy demonstrates activity in refractory ovarian cancer" GYNECOLOGIC ONCOLOGY, ACADEMIC PRESS, LONDON, GB, vol. 102, no. 2, 1 August 2006 (2006-08-01), pages 134-139, XP005542075 ISSN: 0090-8258
- MONK ET AL: "Salvage bevacizumab (rhuMAB VEGF)-based therapy after multiple prior cytotoxic regimens in advanced refractory epithelial ovarian cancer" GYNECOLOGIC ONCOLOGY, ACADEMIC PRESS, LONDON, GB, vol. 102, no. 2, 1 August 2006 (2006-08-01), pages 140-144, XP005542076 ISSN: 0090-8258
- NUMNUM ET AL: "The use of bevacizumab to palliate symptomatic ascites in patients with refractory ovarian carcinoma" GYNECOLOGIC ONCOLOGY, ACADEMIC PRESS, LONDON, GB, vol. 102, no. 3, 1 September 2006 (2006-09-01), pages 425-428, XP005644749 ISSN: 0090-8258
- MONK B J ET AL: "Activity of bevacizumab (rhuMAB VEGF) in advanced refractory epithelial ovarian cancer" GYNECOLOGIC ONCOLOGY, ACADEMIC PRESS, LONDON, GB, vol. 96, no. 3, 1 March 2005 (2005-03-01), pages 902-905, XP004757104 ISSN: 0090-8258
- DATABASE BIOSIS [Online] BIOSCIENCES INFORMATION SERVICE, PHILADELPHIA, PA, US; November 2006 (2006-11), CAMPBELL RICHARD A ET AL: "Anti-VEGF antibody treatment markedly inhibits tumor growth in SCID-hu models of human multiple myeloma." XP002502789 Database accession no. PREV200700257594 & BLOOD, vol. 108, no. 11, Part 1, November 2006 (2006-11), page 254A, 48TH ANNUAL MEETING OF THE AMERICAN-SOCIETY-OF-HEMATOLOGY; ORLANDO, FL, USA; DECEMBER 09 -12, 2006 ISSN: 0006-4971
- FERRARA NAPOLEONE ET AL: "Targeting VEGF-A to treat cancer and age-related macular degeneration" ANNUAL REVIEW OF MEDICINE : SELECTED TOPICS IN THE CLINICALSCIENCES, ANNUAL REVIEWS INC., PALO ALTO, CA, vol. 58, 19 October 2006 (2006-10-19), pages 491-504, XP002479248 ISSN: 0066-4219
- DATABASE MEDLINE [Online] US NATIONAL LIBRARY OF MEDICINE (NLM), BETHESDA, MD, US; July 2001 (2001-07), ANDERSON K C: "Novel biologically based therapies for myeloma." XP002502790 Database accession no. NLM11504280 & CANCER JOURNAL (SUDBURY, MASS.) 2001 JUL-AUG, vol. 7 Suppl 1, July 2001 (2001-07), pages S19-S23, ISSN: 1528-9117
- DATABASE BIOSIS [Online] BIOSCIENCES INFORMATION SERVICE, PHILADELPHIA, PA, US; November 2005 (2005-11), SOMLO GEORGE ET AL: "Phase II randomized trial of bevacizumab versus bevacizumab and thalidomide for relapsed/refractory multiple myeloma." XP002502791 Database accession no. PREV200600184940 & BLOOD, vol. 106, no. 11, Part 1, November 2005 (2005-11), page 723A, 47TH ANNUAL MEETING OF THE AMERICAN-SOCIETY-OF-HEMATOLOGY; ATLANTA, GA, USA; DECEMBER 10 -13, 2005 ISSN: 0006-4971
- CAMPBELL RICHARD A ET AL: "LAGlambda-1: a clinically relevant drug resistant human multiple myeloma tumor murine model that enables rapid evaluation of treatments for multiple myeloma." INTERNATIONAL JOURNAL OF ONCOLOGY JUN 2006, vol. 28, no. 6, June 2006 (2006-06), pages 1409-1417, XP002502962 ISSN: 1019-6439 cited in the application

## Description

### FIELD OF THE INVENTION

The present invention relates to compositions and their use in a method for treating a neoplastic growth, including a relapsed or refractory neoplastic growth using VEGF antagonists as single agents or in combination with other therapies.

### BACKGROUND OF THE INVENTION

Neoplasms are abnormal cell growths, which may be cancerous or benign. Plasma cells (also referred to as plasma B cells or plasmocytes) develop from mature B lymphocytes (B cells), and are normally involved in secreting antibodies in order to fight foreign elements in the body (e.g., bacteria or virus inflections). The presence of plasma cell neoplasms can result in less active healthy red blood cells, white blood cells, and platelets. This condition may cause anemia or easy bleeding, or make it easier to get an infection. The abnormal plasma cells often form tumors in bones or soft tissues of the body. The plasma cell neoplasms may also produce a large amount of a single antibody, called M protein (or monoclonal protein, myeloma protein or paraprotein), that is not needed by the body, does not help fight infection and can cause damage to the kidneys. In some cases, malignant plasma cells lose the ability to make and match heavy chains and light chains so that kappa and lambda light chains (also called Bence Jones protein) leave the cell unattached into the blood and are excreted in the urine. Examples of plasma cell neoplasms include multiple myeloma (MM), solitary plasmacytoma of the bone (SPB), plasma cell leukemias, and extramedullary plasmacytomas (EMP).

Multiple myeloma (MM) is a malignancy characterized by the expansion of plasma B cells that produce monoclonal immunoglobulin (e.g., IgG, IgA, IgD, IgE, or free kappa or lamda light chains). The overall survival of patients with MM varies greatly from a few months to many years; the mean is approximately five years. Anemia, hypercalcemia and bone lesions correlate directly with total mass of myeloma cells and have important prognostic significance. Other prognostic factors include age, the plasma cell labeling index, serum beta2-microglobulin, C-reactive protein, thymidine kinase, and soluble interleukin-6 receptor. Major complications, such as infection and renal insufficiency, are the most common causes of death for myeloma patients. Almost all patients with multiple myelomas have a risk of eventual relapse (Kyle, RK et al., (2004) N Engl J Med 351:1860-1873).

Siegel et al. (2006) Blood 108(11, part 1): 1022A describes a phase II trial of SCIO-469 as monotherapy or in combination with bortezornib in relapsed refractory multiple myeloma.

Richardson et al. (2003) Blood 102(11): 235a describes a multi-center, randomized, phase II study to evaluate the efficacy and safety of 2 CDC-5013 dose regimens when used alone or in combination with dexamethasone for the treatment of relapsed or refractory multiple myeloma.

### SUMMARY OF THE INVENTION

The present invention provides means, as defined in the claims, for treating a neoplasm in a patient relapsed from or refractory to bortezomib therapy comprising the step of administering a VEGF antagonist. Also disclosed is a method for treating a plasma cell neoplasm in a patient relapsed for or refractory to an alkylating agent therapy comprising the step of administering a VEGF antagonist. Also disclosed is a method for treating a neoplasm in a relapsed or refractory patient comprising the step of administering a VEGF antagonist and a proteasome inhibitor.

Also disclosed is a method for treating a neoplasm in a relapsed or refractory patient comprising the step of administering a VEGF antagonist and thalidomide or a thalidomide analogue.

According to one embodiment, the relapsed or refractory patient is further administered a thalidomide or a thalidomide analogue. According to another embodiment, the relapsed or refractory patient is further administered a proteasome inhibitor. According to another embodiment, the relapsed or refractory patient is administered the VEGF antagonist as a single agent.

According to yet another embodiment, the the relapsed or refractory patient is further administered an additional therapeutic agent. According to one embodiment, the additional therapeutic agent is selected from the group consisting of an alkylating agent, a steroid, bisphosphonates and a proteasome inhibitor. According to another embodiment, the additional therapeutic agent is selected from the group consisting of melphalan, prednisone, thalidomide, andriamycin (doxorubicin), doxorubicin HCL liposome injection (Doxil®), bortezomib (velcade®), lenalidomide (CC-5013, Revlimid®), dexamethasone, vincristine (Oncovin®), carmustine and cyclophasphamide (cytoxan).

In yet another embodiment, the the relapsed or refractory patient is further administered a combination therapy. In one embodiment, the combination therapy is selected from the group consisting of: melphalan/prednisone combination (MP), melphalan/prednisone/thalidomide combination (MPT), doxil/bortezomib combination, thalidomide/dexamethasone combination (TD), bortezomib/doxorubicin/thalidomide/dexamethasone combination (BATD), bortezomib/thalidomide combination (BT), bortezomib/melphalan/dexamethasone/thalidomide combination (BMDT), bortezomib/melphalan/prednisone/thalidomide (BMPT) combination, bortezomib/peglyated liposomal doxorubicin/thalidomide combination (BTD), bortezomib/cyclophasphamide/prednisone (BCP), vincristine/carmustine/melphalan/cyclophosphamide/prednisone combination (VBMCP) and vincristine/doxorubicin/dexamethasone combination (VAD).

In accordance with the invention, the proteasome inhibitor is bortezomib. In one embodiment, the alkylating agent is melaphalan.

According to the invention, the plasma cell neoplasm is multiple myeloma.

According to the invention, the VEGF antagonist is an anti-VEGF antibody. According to one embodiment, the anti-VEGF antibody is a human or humanized anti-VEGF antibody. In one preferred embodiment, the anti-VEGF antibody is the AVASTIN^{®} antibody.

Compositions comprising the combinations of therapeutic agents provided in this invention are also disclosed. In one embodiment, a composition comprises a VEGF antagonist and a proteosome inhibitor. In another embodiment, the composition further comprises another therapeutic agent selected from the group consisting of an alkylating agent, a steroid, bisphosphonates and a different VEGF antagonist. Use of VEGF antagonists, alone or in combination with other therapeutic agents described herein, in the manufacture of a medicament to treat the indications provided herein are also provided, as defined in the claims.

### BRIEF DESCRIPTION OF THE DRAWINGS

Figure 1 shows the human IgG levels in a mouse after implantation of a LAGk-1A tumor and then treatment with an anti-VEGF antibody: (A) tabular form and (B) graphic form.
Figure 2 shows the tumor volume of a LAGk-1A tumor in a mouse after implantation of the tumor and then treatment with an anti-VEGF antibody: (A) tabular form and (B) graphic form.
Figure 3 shows the human IgG levels in a mouse after implantation of a LAGk-1B tumor and then treatment with an anti-VEGF antibody: (A) tabular form and (B) graphic form.
Figure 4 shows the tumor volume of a LAGk-1B tumor in a mouse after implantation of the tumor and then treatment with an anti-VEGF antibody: (A) tabular form and (B) graphic form.
Figure 5A shows the human IgG levels in a mouse after implantation of a LAGk-1B tumor and then treatment with an anti-VEGF antibody at day 21.
Figure 5B shows the tumor volume of a LAGk-1B tumor in a mouse after implantation of the tumor and then treatment with an anti-VEGF antibody at day 21.

### DETAILED DESCRIPTION OF THE INVENTION

### DEFINITIONS

Proteasomes are multisubunit, cylindrical proteases found in eukaryotes, eubacteria, and archaebacteria. The proteasome's active sites face a central chamber buried within the cylindrical particle. Eukaryotic proteasomes come in two sizes, the 20S proteasome and the considerably larger ATP-dependent 26S proteasome. The latter is formed when the 20S proteasome binds one or two multisubunit ATPase-containing particles known as 19S regulatory complexes. The 26S proteasome is responsible for degrading ubiquitylated proteins and is therefore essential for a vast array of cellular processes including cell-cycle traverse, control of transcription, regulation of enzyme levels, and apoptosis. Both 20S and 26S proteasomes can associate with other protein complexes.

"Proteasome inhibitor" refers to an agent that inhibits a protease activity of a proteasome (e.g., any one or all of chymotryptic, tryptic and peptidylglutamylpeptide hydrolyzing activities). According to one preferred embodiment the proteasome is the human 20S and/or 26S proteasome. Examples of proteasome inhibitors include bortezomib, MG132, lactacystin, epoxomicin, and salinosporamide A (NPI-0052, Nereus Pharmaceutical) See, e.g., structures disclosed in Voorhees, PM et.a.l., (2006) 46:189-213, peptide aldehydes WO 95/24914, WO 91/13904, Iqbal et al. (1995) J. Med. Chem. 38:2276-2277, peptide boronic acids WO 96/13266 , lactacystin, and lactacystin analogs (Fenteany et al. (1994) Proc. Natl. Acad. Sci. USA 91:3358; WO 96/32105) and salinosporamide A (Macheria, VR, et al., (2005) 48:3684-7.

The term "VEGF" or "VEGF" as used herein refers to the 165-amino acid human vascular endothelial cell growth factor and related 121-, 189-, and 206- amino acid human vascular endothelial cell growth factors, as described by Leung et al. Science, 246:1306 (1989), and Houck et al. Mol. Endocrin., 5:1806 (1991), together with the naturally occurring allelic and processed forms thereof. The term "VEGF" also refers to VEGFs from non-human species such as mouse, rat or primate. Sometimes the VEGF from a specific species are indicated by terms such as hVEGF for human VEGF, mVEGF for murine VEGF, and etc. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "VEGF₁₆₅." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

A "VEGF antagonist" refers to a molecule capable of neutralizing, blocking, inhibiting, abrogating, reducing or interfering with VEGF activities including its binding to one or more VEGF receptors. VEGF antagonists include anti-VEGF antibodies and antigen-binding fragments thereof, aptamer, receptor molecules and derivatives which bind specifically to VEGF thereby sequestering its binding to one or more receptors, anti-VEGF receptor antibodies and VEGF receptor antagonists such as small molecule inhibitors of the VEGFR tyrosine kinases. Examples of VEGF antagonists include, but are not limited to, FLT-Fc, KDR-Fc, FLT/KDR-Fc, vatalanib (PTK-787/ZK222584), SU-5416 (semaxanib), SU-6668, SU-11248, SU-14813, AZD-6474, AZD-2171, CGP-41251, CEP-5214, BIBF1000, VGA1102, CP-547632, CEP-7055, AG-013736, IM-842 (a dipeptide ofL-Glutamyl and L-Tryptophan) or GW-786034, as well as those described in WO 02/36564, WO 99/52869, WO 00/18734, WO 00/73297, WO 01/27080, WO 01/27081, WO 01/32651, WO 01/60814, WO 99/48868 and WO 98/35958.

An "anti-VEGF antibody" is an antibody that binds to VEGF with sufficient affinity and specificity. Preferably, the anti-VEGF antibody of the invention can be used as a therapeutic agent in targeting and interfering with diseases or conditions wherein the VEGF activity is involved. An anti-VEGF antibody will usually not bind to other VEGF homologues such as VEGF-B or VEGF-C, nor other growth factors such as PIGF, PDGF or bFGF. A preferred anti-VEGF antibody is a monoclonal antibody that binds to the same epitope as the monoclonal anti-VEGF antibody A4.6.1 produced by hybridoma ATCC HB 10709. More preferably the anti-VEGF antibody is a recombinant humanized anti-VEGF monoclonal antibody generated according to Presta et al. (1997) Cancer Res. 57:4593-4599, including but not limited to the antibody known as bevacizumab (BV; AVASTIN^{®} antibody).

The anti-VEGF antibody "bevacizumab (BV)", also known as "rhuMAb VEGF" or "AVASTIN^{®}, is a recombinant humanized anti-VEGF monoclonal antibody generated according to Presta et al. (1997) Cancer Res. 57:4593-4599. It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of bevacizumab, including most of the framework regions, is derived from human IgG1, and about 7% of the sequence is derived from the murine antibody A4.6.1. Bevacizumab has a molecular mass of about 149,000 daltons and is glycosylated.

"Refractory" refers to the resistance or non-responsiveness of a disease or condition to a treatment (e.g., the number of neoplastic plasma cells increases even though treatment if given). Unless otherwise indicated, the term "refractory" refers a resistance or non-responsiveness to any previous treatment including, but not limited to, chemotherapies and stem cell transplantation treatments.

"Relapsed" refers to the regression of the patient's illness back to its former diseased state, especially the return of symptoms following an apparent recovery or partial recovery. Unless otherwise indicted, relapsed state refers to the process of returning to or the return to illness before the previous treatment including, but not limited to, chemotherapies and stem cell transplantation treatments.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma, nasopharyngeal cancer, glioma and various types of head and neck cancer, as well as hematological malignancies such as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma (MCL); AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; multiple myeloma and post-transplant lymphoproliferative disorder (PTLD).

"B cell neoplasm" refers to an abnormal B cell growth which may be cancerous or benign and which often has uncontrolled cell division. B cell neoplasm include, but are limited to, plasma cell neoplasms, Hodgkin's disease including lymphocyte predominant Hodgkin's disease (LPHD); non-Hodgkin's lymphoma (NHL); follicular center cell (FCC) lymphomas; acute lymphocytic leukemia (ALL); chronic lymphocytic leukemia (CLL); Hairy cell leukemia and CD20-positive neoplasms. The non-Hodgkins lymphoma include low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, plasmacytoid lymphocytic lymphoma, mantle cell lymphoma, AIDS-related lymphoma and Waldenstrom's macroglobulinemia. Treatment of relapses of these cancers are also contemplated. LPHD is a type of Hodgkin's disease that tends to relapse frequently despite radiation or chemotherapy treatment. CLL is one of four major types of leukemia. A cancer of mature B-cells called lymphocytes, CLL is manifested by progressive accumulation of cells in blood, bone marrow and lymphatic tissues. Indolent lymphoma is a slow-growing, incurable disease in which the average patient survives between six and 10 years following numerous periods of remission and relapse.

The term "non-Hodgkin's lymphoma" or "NHL", as used herein, refers to a cancer of the lymphatic system other than Hodgkin's lymphomas. Hodgkin's lymphomas can generally be distinguished from non-Hodgkin's lymphomas by the presence of Reed-Sternberg cells in Hodgkin's lymphomas and the absence of said cells in non-Hodgkin's lymphomas. Examples of non-Hodgkin's lymphomas encompassed by the term as used herein include any that would be identified as such by one skilled in the art (e.g., an oncologist or pathologist) in accordance with classification schemes known in the art, such as the Revised European-American Lymphoma (REAL) scheme as described in Color Atlas of Clinical Hematology, Third Edition; A. Victor Hoffbrand and John E. Pettit (eds.) (Harcourt Publishers Limited 2000) (see, in particular Fig. 11.57, 11.58 and/or 11.59). More specific examples include, but are not limited to, relapsed or refractory NHL, front line low grade NHL, Stage III/IV NHL, chemotherapy resistant NHL, precursor B lymphoblastic leukemia and/or lymphoma, small lymphocytic lymphoma, B cell chronic lymphacytic leukemia and/or prolymphocytic leukemia and/or small lymphocytic lymphoma, B-cell prolymphocytic lymphoma, immunocytoma and/or lymphoplasmacytic lymphoma, marginal zone B cell lymphoma, splenic marginal zone lymphoma, extranodal marginal zone - MALT lymphoma, nodal marginal zone lymphoma, hairy cell leukemia, plasmacytoma and/or plasma cell myeloma, low grade/follicular lymphoma, intermediate grade/follicular NHL, mantle cell lymphoma, follicle center lymphoma (follicular), intermediate grade diffuse NHL, diffuse large B-cell lymphoma, aggressive NHL (including aggressive front-line NHL, and aggressive relapsed NHL), NHL relapsing after or refractory to autologous stem cell transplantation, primary mediastinal large B-cell lymphoma, primary effusion lymphoma, high grade immunoblastic NHL, high grade lymphoblastic NHL, high grade small non-cleaved cell NHL, bulky disease NHL, Burkitt's lymphoma, precursor (peripheral) T-cell lymphoblastic leukemia and/or lymphoma, adult T-cell lymphoma and/or leukemia, T cell chronic lymphocytic leukemia and/or prolymphacytic leukemia, large granular lymphocytic leukemia, mycosis fungoides and/or Sezary syndrome, extranodal natural killer/T-cell (nasal type) lymphoma, enteropathy type T-cell lymphoma, hepatosplenic T-cell lymphoma, subcutaneous panniculitis like T-cell lymphoma, skin (cutaneous) lymphomas, anaplastic large cell lymphoma, angiocentric lymphoma, intestinal T cell lymphoma, peripheral T-cell (not otherwise specified) lymphoma and angioimmunoblastic T-cell lymphoma.

"Plasma cell neoplasm" as used herein refers to an abnormal plasma cell growth, which may be cancerous or benign and which often has uncontrolled cell division. Examples of plasma cell neoplasms include multiple myeloma (MM), plasmocytoma (e.g., extramedullary plasmacytomas (EMP), solitary plasmacytoma of the bone (SPB), malignant plasmacytoma), macroglobulinemia (e.g., Waldenström's Macroglobulinemia (Lymphoplasmacytic Leukemia), monoclonal gammopathy of undetermined significance (MGUS), plasma cell leukemia, hyperglobulinemic purpura and kahler disease.

Multiple myeloma can be characterized by, *inter alia,* skeletal destruction, renal failure, anemia and hypercalcemia. Common symptoms of MM include fatigue, bone pain, and recurrent infections. As the number of myeloma cells increases, fewer red blood cells, white blood cells, and platelets are made. The myeloma cells also damage and weaken the hard parts of the bones. However, sometimes multiple myeloma does not cause any symptoms. In rare cases, multiple myeloma can cause organs to fail. This may be caused by a condition called amyloidosis. Antibody proteins build up and may bind together and collect in organs, such as the kidney and heart. This can cause the organs to become stiff and unable to function.

Plasmacytoma is a type of plasma cell neoplasm, the abnormal plasma cells (myeloma cells) collect in one location and form a single tumor. A plasmacytoma may form in bone marrow or may be extramedullary (in soft tissues outside of the bone marrow). Plasmacytoma of the bone often becomes multiple myeloma. Extramedullary plasmacytomas commonly form in tissues of the throat and sinuses.

Macroglobulinemia is an abnormal plasma cells built up in the bone marrow, lymph nodes, and/or spleen. The cells make too much M protein, which causes the blood to become thick. The lymph nodes, liver, and spleen may become swollen. The thickened blood may cause problems with blood flow in small blood vessels. Symptoms of macroglobulinemia depend on the part of the body affected. Most patients with macroglobulinemia have no symptoms. In this type of plasma cell neoplasm, there are abnormal plasma cells in the bone marrow but there is no cancer. The abnormal plasma cells produce M protein that may be found during a routine blood or urine test. In most patients, the amount of M protein stays the same and there are no symptoms or problems. In some patients, MGUS may later become a more serious condition, such as multiple myeloma or lymphoma.

Waldenström's macroglobulinemia (WM) is a rare, indolent (slow-growing) non-Hodgkin's lymphoma (cancer that begins in the cells of the immune system). WM is also called lymphoplasmacytic lymphoma. WM starts in plasma cells, which develop from white blood cells called B lymphocytes or B cells. Macroglobulinemia occurring in older persons, especially women, characterized by anemia, hyperglobulinemia, and the proliferation of cells resembling white blood cells or plasma cells in the bone marrow. In WM, abnormal plasma cells multiply out of control, producing large amounts of a protein called monoclonal macroglobulin (IgM) antibody. High levels of IgM in the blood cause hyperviscosity (thickness or gumminess), which leads to many of the symptoms of Waldenström's.

Examples of therapeutic agents and combinations of therapeutic agents useful according to this invention include the AVASTIN^{®} antibody, bisphosphosphonates, thalidomide analogues, bortezomib, melphalan; prednisone; thalidomide; andriamycin (doxorubicin); doxorubicin HCL liposome injection (Doxil®), bortezomib (velcade®); lenalidomide (CC-5013, Revlimid®); dexamethasone; vincristine (Oncovin®), carmustine, cyclophasphamide (cytoxan), melphalan/prednisone/thalidomide combination (MPT); thalidomide/dexamethasone (TD); bortezomib/doxorubicin/thalidomide/dexamethasone combinations (BATD); bortezomib/melphalan/dexamethasone/thalidomide combinations (BMDT); bortezomib/melphalan/prednisone/thalidomide (BMPT) combinations; bortezomib/peglyated liposomal doxorubicin/thalidomide combinations (BTD), and other alkylating agent combination therapies and corticosteroid agent combination therapies.

Alkylating agents include, but are not limited to, melphalan and cyclophasphamide. An example of an alkylating agent combination therapy includes, but is not limited to, Vincristine, Carmustine, melphalan, cyclophosphamide, prednisone (VBMCP).

"Steroids" include, but are not limited to, corticorticosteroids such as dexamethasone and prednisone. An example of a corticosteroid agent combination therapy, includes but is not limited to, vincristine/doxorubicin/dexamethasone (VAD).

"Bisphosphonates" include, but are not limited to, pamidronate (e.g., Aredia® & others), zoledronic acid (e.g., Zometa®) and clodronate (e.g., Bonefos®) (clodronate).

"Thalidomide analogues" refer to immunomodulatory analogues of thalidomide, including but not limited to, lenalidomide (Revlimid®), CC-4047 (Actimid™), CC11006, ENMD-0995, and CC11006. See also, Machado, AL et al., (2002) Bioorganic & Med. Chem. Lets. 15:1169-1172; Lepper, ER et al., (2004)) J. Med. Chem. 47:2219-2217; US Patent Nos. 7,719,106 and 7,041,680.

"Proteasome inhibition therapy" refers to any therapeutic treatment comprising the step of administering a proteasome inhibitor.

"Alkylating agent therapy" refers to any therapeutic treatment comprising the step of administering an alkylating agent.

"Thalidomide or thalidomide analogue therapy" refers to any therapeutic treatment comprising the step of administering a thalidomide or thalidomide analogue, respectively.

"Chronic" administration refers to administration of the agent(s) in a continuous mode as opposed to an acute mode, so as to maintain the initial therapeutic effect (activity) for an extended period of time.

"Intermittent" administration is treatment that is not consecutively done without interruption, but rather is cyclic in nature.

Administration "in combination with" one or more further therapeutic agents includes simultaneous and consecutive administration of the therapeutic agents in any order.

An "effective amount" of a therapeutic agent as disclosed herein is an amount sufficient to carry out a specifically stated purpose. An "effective amount" can be determined empirically and by a variety of methods relating to the stated purpose.

The term "therapeutically effective amount" refers to an amount of a therapeutic agent of this invention effective to "treat" a disease or disorder in a mammal (aka patient). In the case of cancer, the therapeutically effective amount of the drug can reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See the definition herein of "treating". To the extent the drug can prevent growth and/or kill existing cancer cells, it can be cytostatic and/or cytotoxic. The therapeutically effective amount can be a growth inhibitory amount or a cytotoxic amount depending on the therapeutic agent and the disease to be treated.

"Treating" or "treatment" or "alleviation" refers to therapeutic meaures, wherein the object is to prevent or slow down (lessen) the targeted pathologic condition or disorder. Those in need of treatment include those already with the disorder as well as those prone to have the disorder or those in whom the disorder is to be prevented. These terms indicate the therapeutic and prophylactic uses herein are successful if they ameliorate, lessen or decrease the symptoms, complications or other problems associated with a disease or ameliorate, lessen or decrease the chance of onset or frequency of the symptoms, complications or other problems associated with a disease.

A "growth inhibitory amount" of a therapeutic agent of this invention is an amount capable of inhibiting the growth of a cell, especially tumor, e.g., cancer cell, either *in vitro* or *in vivo.* A "growth inhibitory amount" of a therapeutic agent of this invention for purposes of inhibiting neoplastic cell growth can be determined empirically and by known methods or by examples provided herein.

A "cytotoxic amount" of a therapeutic agent of this invention is an amount capable of causing the destruction of a cell, especially tumor, e.g., cancer cell, either *in vitro* or *in vivo.* A "cytotoxic amount" for purposes of inhibiting neoplastic cell growth can be determined empirically and by methods known in the art.

The term "antibody" is used in the broadest sense and specifically covers, for example, single monoclonal antibodies (including agonist, antagonist, and neutralizing antibodies), antibody compositions with polyepitopic specificity, polyclonal antibodies, single chain anti-antibodies, and fragments of antibodies (see below) as long as they specifically bind a native polypeptide and/or exhibit a biological activity or immunological activity of this invention. According to one embodiment, the antibody binds to an oligomeric form of a target protein, e.g., a trimeric form. According to another embodiment, the antibody specifically binds to a protein, which binding can be inhibited by a monoclonal antibody of this invention (e.g., a deposited antibody of this invention, etc.). The phrase "functional fragment or analog" of an antibody is a compound having a qualitative biological activity in common with an antibody to which it is being referred. For example, a functional fragment or analog of an antibody of this invention can be one which can specifically bind to VEGF. In one embodiment, the antibody can prevent or substantially reduce the ability of VEGF to induce cell proliferation. The term "immunoglobulin" (Ig) is used interchangeably with "antibody" herein.

An "isolated antibody" is one which has been identified and separated and/or recovered from a component of its natural environment. Contaminant components of its natural environment are materials which would interfere with diagnostic or therapeutic uses for the antibody, and can include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The basic 4-chain antibody unit is a heterotetrameric glycoprotein composed of two identical light (L) chains and two identical heavy (H) chains (an IgM antibody consists of 5 of the basic heterotetramer unit along with an additional polypeptide called J chain, and therefore contain 10 antigen binding sites, while secreted IgA antibodies can polymerize to form polyvalent assemblages comprising 2-5 of the basic 4-chain units along with J chain). In the case of IgGs, the 4-chain unit is generally about 150,000 daltons. Each L chain is linked to a H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ε isotypes. Each L chain has at the N-terminus, a variable domain (V_{L}) followed by a constant domain (C_{L}) at its other end. The V_{L} is aligned with the V_{H} and the C_{L} is aligned with the first constant domain of the heavy chain (C_{H}1). Particular amino acid residues are believed to form an interface between the light chain and heavy chain variable domains. The pairing of a V_{H} and V_{L} together forms a single antigen-binding site. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa and lambda, based on the amino acid sequences of their constant domains. Depending on the amino acid sequence of the constant domain of their heavy chains (C_{H}), immunoglobulins can be assigned to different classes or isotypes. There are five classes of immunoglobulins: IgA, IgD, IgE, IgG, and IgM, having heavy chains designated α, δ, γ, ε, and µ, respectively. The γ and α classes are further divided into subclasses on the basis of relatively minor differences in C_{H} sequence and function, e.g., humans express the following subclasses: IgG1, IgG2, IgG3, IgG4, IgA1, and IgA2.

The term "variable" refers to the fact that certain segments of the variable domains differ extensively in sequence among antibodies. The V domain mediates antigen binding and define specificity of a particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a *-sheet configuration, connected by three hypervariable regions, which form loops connecting, and in some cases forming part of, the *-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the V_{L}, and around about 1-35 (H1), 50-65 (H2) and 95-102 (H3) in the V_{H} (in one embodiment, H1 is around about 31-35); Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the V_{L}, and 26-32 (H1), 53-55 (H2) and 96-101 (H3) in the V_{H}; Chothia and Lesk J. Mol. Biol. 196:901-917 (1987)).

The term "monoclonal antibody" as used herein refers to an antibody obtained from a population of substantially homogeneous antibodies, i.e., the individual antibodies comprising the population are identical except for possible naturally occurring mutations that can be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they can be synthesized uncontaminated by other antibodies. The modifier "monoclonal" is not to be construed as requiring production of the antibody by any particular method. For example, the monoclonal antibodies useful in the present invention can be prepared by the hybridoma methodology first described by Kohler et al., Nature, 256:495 (1975), or can be made using recombinant DNA methods in bacterial, eukaryotic animal or plant cells (see, e.g., U.S. Patent No. 4,816,567). The "monoclonal antibodies" can also be isolated from phage antibody libraries using the techniques described in Clackson et al., Nature, 352:624-628 (1991), Marks et al., J. Mol. Biol. , 222:581-597 (1991), and the Examples below, for example.

The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this invention (see U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, Ape etc), and human constant region sequences.

An "intact" antibody is one which comprises an antigen-binding site as well as a C_{L} and at least heavy chain constant domains, C_{H}1, C_{H}2 and C_{H}3. The constant domains can be native sequence constant domains (e.g. human native sequence constant domains) or amino acid sequence variant thereof. Preferably, the intact antibody has one or more effector functions.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments. The expression "linear antibodies" generally refers to the antibodies described in Zapata et al., Protein Eng., 8(10):1057-1062 (1995). Briefly, these antibodies comprise a pair of tandem Fd segments (VH-CH 1-VH-CH1) which, together with complementary light chain polypeptides, form a pair of antigen binding regions. Linear antibodies can be bispecific or monospecific.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H}1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having divalent antigen-binding activity and is still capable of cross-linking antigen. Fab' fragments differ from Fab fragments by having additional few residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by di sulfides. The effector functions of antibodies are determined by sequences in the Fc region, which region is also the part recognized by Fc receptor (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (*e.g*., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "species-dependent antibody" is an antibody which has a stronger binding affinity for an antigen from a first mammalian species than it has for a homologue of that antigen from a second mammalian species. Normally, the species-dependent antibody "bind specifically" to a human antigen (i.e., has a binding affinity (Kd) value of no more than about 1 x 10⁻⁷ M, preferably no more than about 1 x 10⁻⁸and most preferably no more than about 1 x 10⁻⁹ M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

A therapeutic agent of this invention "which binds" a target of interest is one that binds the target with sufficient affinity such that a therapeutic agent is useful as a therapeutic agent in acting on a cell or tissue expressing of having the target(s), and does not significantly cross-react with other targets. In such embodiments, the extent of binding of the therapeutic agent to a "non-target" will be less than about 10% of the binding of the therapeutic agent to its particular target as determined by, e.g., fluorescence activated cell sorting (FACS) analysis or radioimmunoprecipitation (RIA). With regard to the binding of a therapeutic agent to a target molecule, the term "specific binding" or "specifically binds to" or is "specific for" a particular target or an epitope on a particular target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. In one embodiment, the term "specific binding" refers to binding where a molecule binds to a particular target or epitope on a particular target(s) without substantially binding to any other target or epitope.

"Patient" refers to the subject to be treated. In a preferred embodiment, the subject is a mammal.

"mammal" for purposes of treatment refers to any animal classified as a mammal, including humans, domestic and farm animals, nonhuman primates, and zoo, sports, or pet animals, such as dogs, horses, cats, cows, etc.. In one preferred embodiment, the mammal is a human.

Compositions of this invention can comprise one or more therapeutic agent of this invention and a carrier. "Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN®, polyethylene glycol (PEG), and PLURONICS®.

### Dosage

Depending on the indication to be treated and factors relevant to the dosing that a physician of skill in the field would be familiar with, the therapeutic agents of this invention will be administered at a dosage that is efficacious for the treatment of that indication while minimizing toxicity and side effects. For example, a starting dosing regimen for anti-VEGF antibodies can be 5- 10mg/kg every two weeks. In one embodiment, the starting dosage for bortezomib is 1.3mg/m² twice weekly for two weeks followed by a 10 day rest period. In another embodiment, the starting dose of lenalidomide is 10mg once per day. Therapeutic agents of this invention may be administered chronically, intermittently, sequentially or concurrently as needed.

Throughout this specification and claims, the word "comprise," or variations such as "comprises" or "comprising," will be understood to imply the inclusion of a stated integer or group of integers but not the exclusion of any other integer or group of integers.

The foregoing written description is considered to be sufficient to enable one skilled in the art to practice the invention. The following Examples are offered for illustrative purposes only, and are not intended to limit the scope of the present invention in any way. Indeed, various modifications in addition to those shown and described herein will become apparent to those skilled in the art from the foregoing description and may fall within the scope of the appended claims.

### EXAMPLES

### Example 1 - Materials and Methods

Bone marrow biopsies from patients diagnosed with multiple myeloma were obtained. The Ig heavy and light chain isotypes were determined at diagnosis by immunofixation electrophoresis (IFE). One sample, known LAGλ-1 (Los Angeles IgG λ light chain-1), is from an aggressive growing tumor from a patient resistant to melphalan (Campbell, RA et al., (2006) Int. J. Onc. 28:1409-1417). LAGk-1A is from a tumor of a patient sensitive to bortezomib. LAGk-1B is from a tumor of a patient resistant to bortezomib. LAPCLκ-1 is a plasma cell leukemia from a patient.

The anti-VEGF antibodies known as G6-31 used in these studies were previously described (WO 2005/012359). An anti-ragweed mIgG2a was used as a control. Generally, prior to administration, the anti-VEGF MAb was diluted in 100% PBS. Anti-VEGF MAb was administered via intraperitoneal (i.p.) injection bi-weekly at 5mg/kg. The control IgG (e.g., anti-Ragweed antibody) was diluted in 100% PBS prior to administration. The control IgG was be administered via i.p. injection bi-weekly at 5mg/kg.

Determination of human IgG (hIgG) levels. Levels of human IgG subclass 1 were determined by Enzyme-linked immunosorbent assay (ELISA). Human IgA, IgM, IgG, and IgG subclass profile ELISA kits were purchased from Zymed Laboratories (South San Francisco, CA). Mice bearing tumors were bled weakly via retro-orbital bleed. Samples were spun at 13,000 rpm for 30 minutes and serum collected. The IgG subclass 1 ELISA kit was prepared according to the manufacturer's specifications. Absorbance at 450nM with a reference wavelength of 550 nm was determined on a µQuant microplate spectrophotometer with KC Junior software (BioTek Instruments, Winooski, VT).

### Example 2 - Anti-VEGF antibody Single Agent multiple myeloma (MM) studies

Six-to-eight week old male severe combined immunodeficient (SCID) mice were used in this study. Each mouse was surgically implanted with a 2.0-4.0 mm³ LAGk-1A or LAGk-1B into its left hind limb superficial gluteal muscle. The tumors were allowed to grow for 14 days at which time human IgG levels were detectable in the mouse serum, and mice were blindly assigned into one of two treatment groups.

The mice were treated as follows:
LAGκ-1A mice (bortezomib sensitive MM)
Control mlgG2a (5mg/kg, IP biwk)
anti-VEGF MAb G6-31 (5mg/kg IP biwk)

LAGκ-1B mice (bortezomib resistant MM)
Control mIgG2a (5mg/kg IP biwk)
anti-VEGF MAb G6-31 (5mg/kg IP biwk)

Treatment with anti-VEGF antibodies commenced at day 14 or day 21. Generally, the mice were weighed once daily, five times weekly until the termination of the study. Tumor volume was measured bi-weekly using standard calipers. Mice were bled weekly and hIgG levels in the murine serum were measured by ELISA.

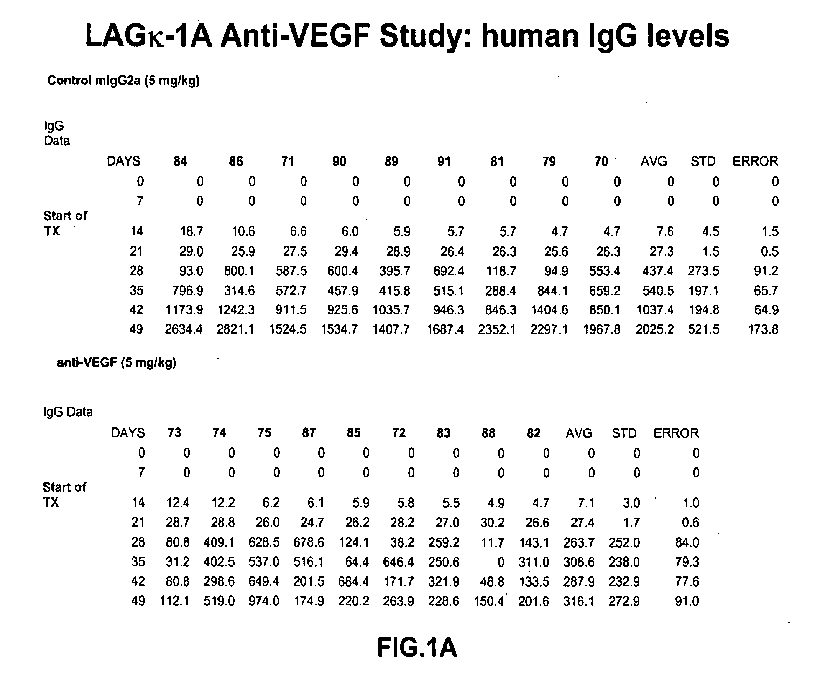

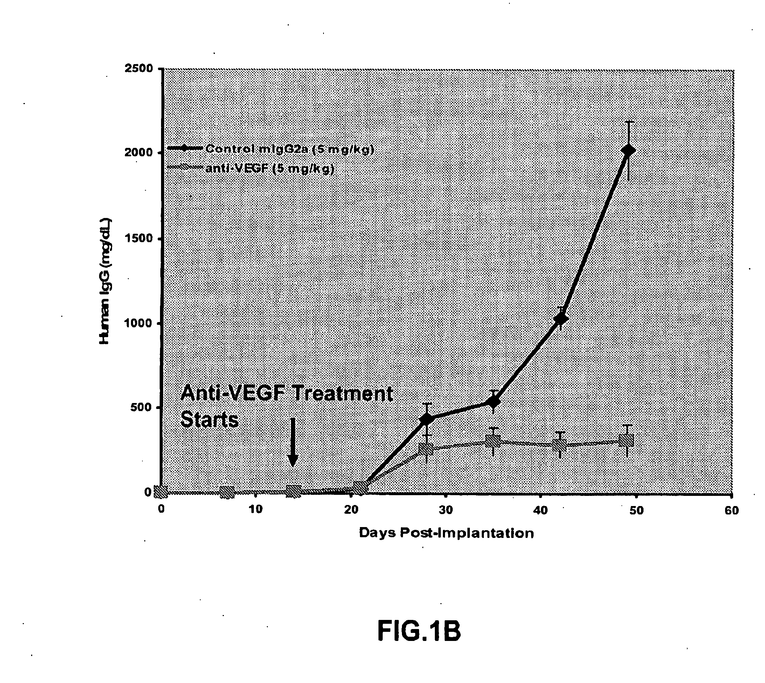

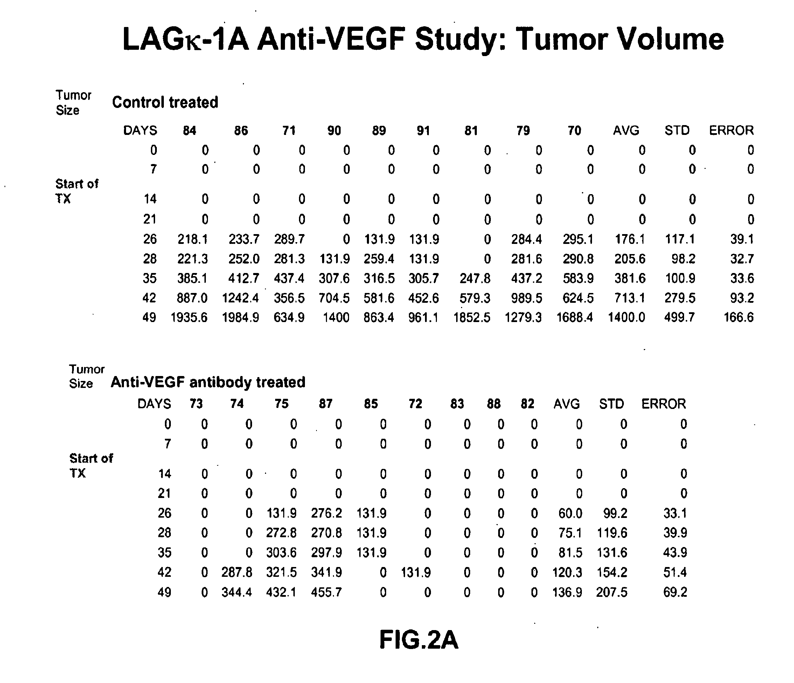

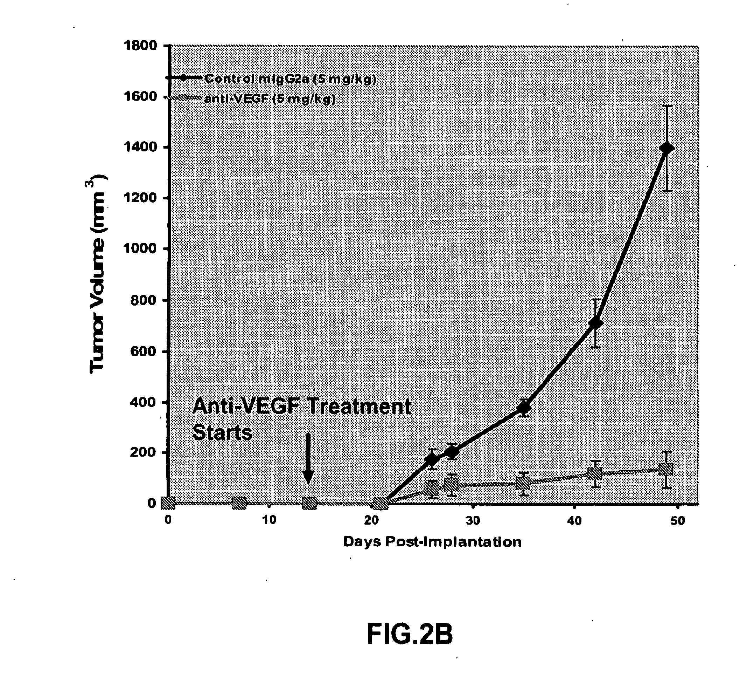

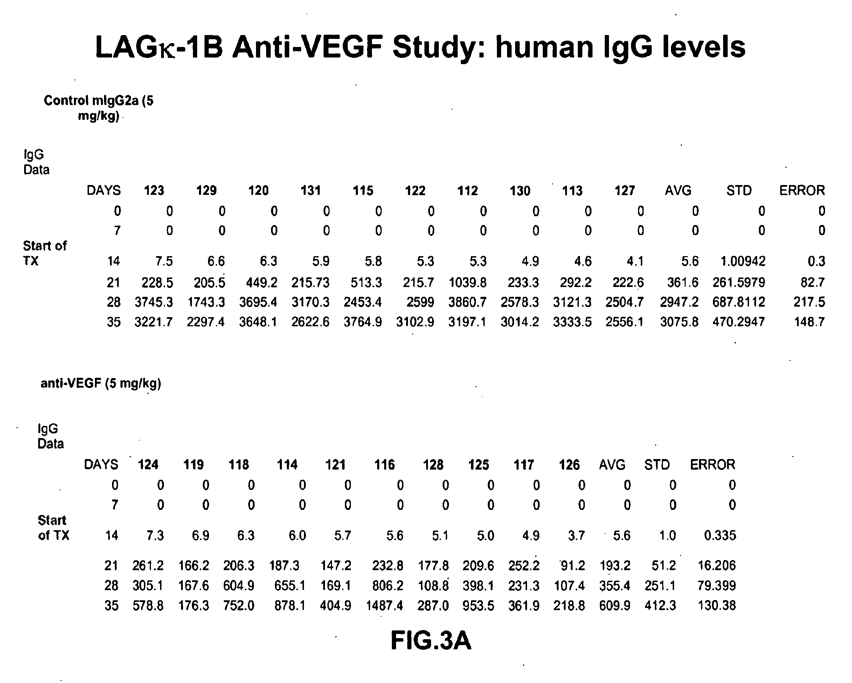

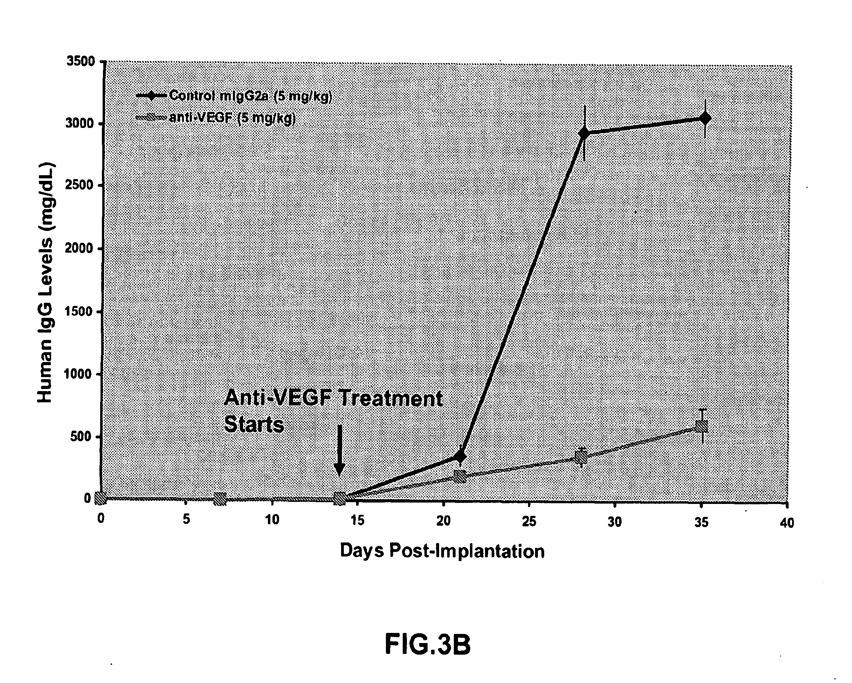

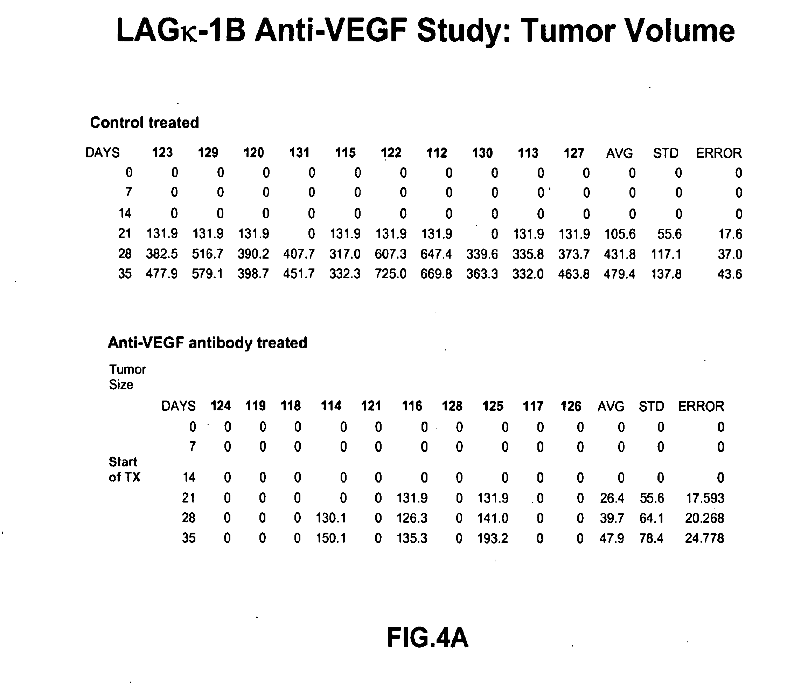

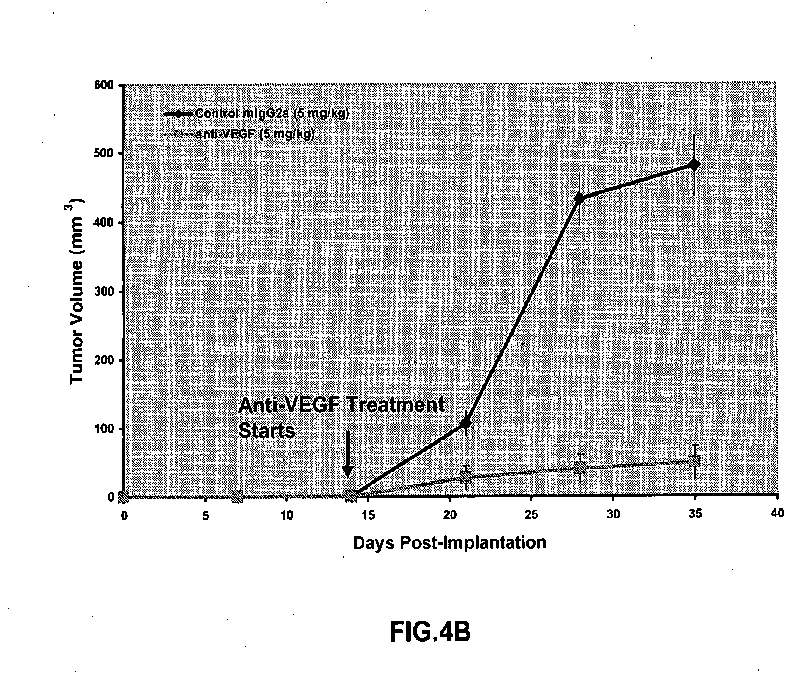

The LAGκ-1A (bortezomib sensitive) tumor results are shown in Figures 1A-B and 2A-B. The LAGκ-1B (bortezomib resistant) tumor results are shown in Figures 3A-B, 4A-Band 5A-B. The mice that received the anti-VEGF antibody showed marked inhibition of tumor growth (p = 0.0005) and reduction of paraprotein levels (p = 0.0002) compared to mice receiving control antibody. On day 42, LAGκ-1A-bearing mice receiving the anti-VEGF antibody showed a 70% reduction in human paraprotein levels and an 80% decrease in tumor volume compared to the control antibody treated animals. Treatment with the anti-VEGF antibody was not associated with any observed toxicity.

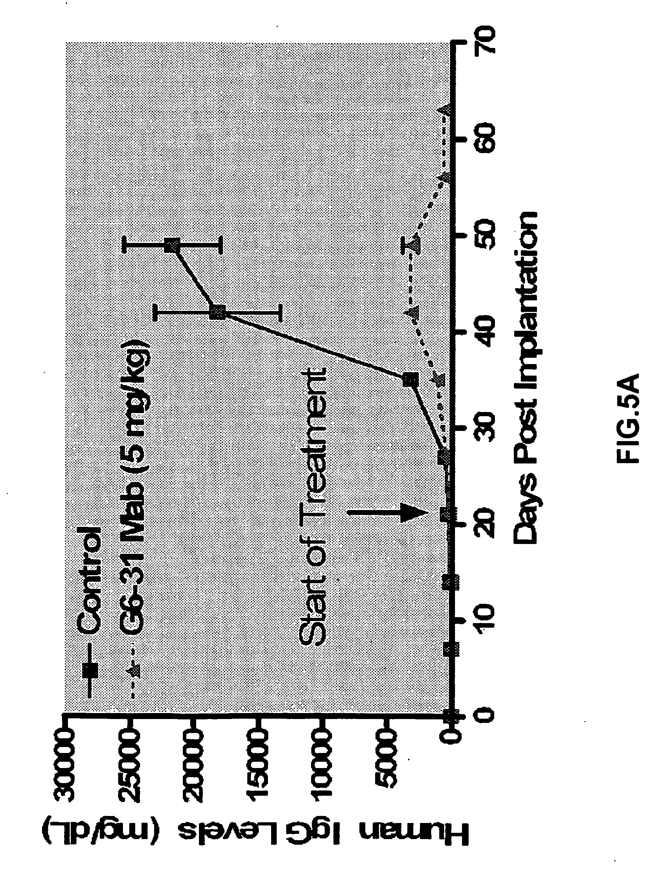

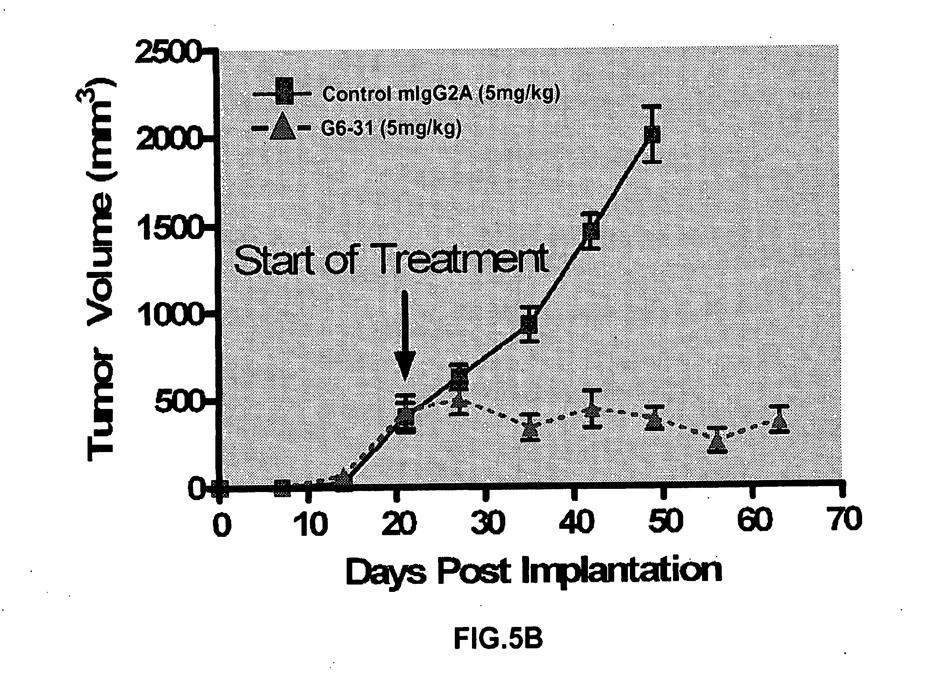

Noteably, the LAGκ-1B-bearing mice (bortezomib resistant) receiving the anti-VEGF antibody showed a substantial reduction in human paraprotein levels and in tumor volume compared to the control antibody treated animals. Thus, anti-VEGF antibody as a single agent was efficacious in reducing bortezomib sensitive and resistant tumors in this study. When the tumor was allowed to grow for 21 days, the tumor volume was sizable before treatment with the anti-VEGF antibody. Figures 5A-B shows that anti-VEGF antibody as a single agent was efficacious in reducing human 1gG1 I levels and LAGκ-1B tumor volume despite the tumor's increased size.

### Example 3 - Anti-VEGF/Bortezomib Combination Studies

SCID mice carrying MM/PCL tumors as described in Example 1 will be randomly assigned into treatment groups (n = 10 mice/group) when the tumors reach an average size of ~100 mm3. Treatment will commence when mice are randomized into their appropriate treatment groups.

Bortezomib will be diluted in 0.9% normal saline at the appropriate dose prior to administration. Bortezomib will be administered via intravenous (i.v.) injection three times weekly at 0.1 mg/kg. In contrast, as a control, the small molecular inhibitor vehicle (SMIv) will be prepared in 0.5% methylcellulose: 0.2% Tween 80: 99.3% diluent. SMIv will be administered via oral gavage daily at a dose to be determined.

The mice will be treated as follows:

| **Group** | **Drug** | **Dose** | **Route** | **# of Mice** | **Schedule** |
|---|---|---|---|---|---|
| 1 | Control mIgG2a | 5 mg/kg | i.p. | 10 | QD x2 days/week |
| 2 | Vehicle (bortezomib) | | i.v. | 10 | QD x2 days/week |
| 3 | Bortezomib | 0.25 mg/kg | i.v. | 10 | QD x2 days/week |
| 4 | Bortezomib | 0.5 mg/kg | i.v. | 10 | QD x2 days/week |
| 5 | Anti-VEGF antibody | 5 mg/kg | i.p. | 10 | QD x2 days/week |
| 6 | Bortezomib + | 0.25 mg/kg | i.v. | 10 | QD x2 days/week |
| | Anti-VEGF antibody | 5 mg/kg | i.p. | | QD x2 days/week |
| 7 | Bortezomib + | 0.5 mg/kg | i.v. | 10 | QD x2 days/week |
| | Anti-VEGF antibody | 5 mg/kg | i.p. | | QD x2 days/week |

The mice will be weighed once daily, five times weekly until the termination of the study. Tumor volumes will be measured bi-weekly using standard calipers. The mice will be bled weekly and hIgG levels in the murine serum will be measured by ELISA. The mean values of tumor volume, tumor weight, and hIgG levels will be included in each group. A comparison will be made between vehicles vs. monotherapy therapy vs. combination therapy.

### Example 4 - Anti-VEGFBortezomib/Lenalidomide Combination Studies

SCID mice carrying MM/PCL tumors as described in Example 1 will be randomly assigned into treatment groups (n = 10 mice/group) when the tumors reach an average size of ~100 mm3. Treatment will commence when mice are randomized into their appropriate treatment groups.

Bortezomib will be diluted in 0.9% normal saline at the appropriate dose prior to administration. Bortezomib will be administered via intravenous (i.v.) injection three times weekly at 0.1 mg/kg. In contrast, as a control, the small molecular inhibitor vehicle (SMIv) will be prepared in 0.5% methylcellulose: 0.2% Tween 80: 99.3% diluent. SMIv will be administered via oral gavage daily at a dose to be determined.

Lenalidomide will be dissolved in DMSO at 10mg/ml. The stock solution will be diluted in sterile 0.9% saline solution to a concentration of1mg/ml. The final concentration of Lenalidomide in all experiments will be less than 0.01%.

The mice will be treated as follows:

| Group | Drug | Dose | Route | # | Schedule |
|---|---|---|---|---|---|
| 1 | Control mIgG2a | 5 mg/kg | i.p. | 10 | 2 days/wk |
| 2 | Vehicle (lenalidomide) | n/a | i.p. | 10 | 5 days/wk |
| 3 | Vehicle (bortezomib) | n/a | i.p. | 10 | 2 days/wk |
| 4 | Lenalidomide | 50 mg/kg | i.p. | 10 | 5 days/wk |
| 5 | Bortezomib | 0.25 mg/kg | i.p. | 10 | 2 days/wk |
| 6 | Bortezomib | 0.5 mg/kg | i.p. | 10 | 2 days/wk |
| 7 | Lenalidomide + Anti-VEGF antibody | 50 mg/kg | i.p. | 10 | 5 days/wk |
| | | 5 mg/kg | | | |
| | | | | | 2 days/wk |
| 8 | Anti-VEGF antibody alone | 5 mg/kg | i.p. | 10 | 2 days/wk |
| 9 | Bortezomib | 0.25 mg/kg | i.p. | 10 | 2 days/wk |
| | Anti-VEGF antibody | 5 mg/kg | | | |
| | | | | | 2 days/wk |
| 10 | Bortezomib + Anti-VEGF antibody | 0.5 mg/kg | i.p. | 10 | 2 days/wk |
| | | 5 mg/kg | | | |
| | | | | | 2 days/wk |

The mice will be weighed once daily, five times weekly until the termination of the study. Tumor volumes will be measured bi-weekly using standard calipers. The mice will be bled weekly and hIgG levels in the murine serum will be measured by ELISA. The mean values of tumor volume, tumor weight, and hIgG levels will be included in each group. A comparison will be made between vehicles vs. monotherapy therapy vs. combination therapy.

## Claims

1. An antagonist anti-VEGF antibody for use in a method for treating multiple myeloma in a patient relapsed from or refractory to bortezomib therapy.

2. The anti-VEGF antibody for use in a method for treating multiple myeloma according to claim 1 wherein the relapsed or refractory patient is administered the anti-VEGF antibody as a single agent.

3. The anti-VEGF antibody for use in a method for treating multiple myeloma according to claim 1 or claim 2, wherein the anti-VEGF antibody is selected from the group consisting of a Fab, Fv, F(ab')2, a scFV, a diabody and a bispecific antibody.

4. The anti-VEGF antibody for use in a method for treating multiple myeloma according to claim 1 or 2 wherein the anti-VEGF antibody is a human or humanized anti-VEGF antibody.

5. The anti-VEGF antibody for use in a method for treating multiple myeloma according to claim 1 or 2 wherein the anti-VEGF antibody is bevacizumab.

6. Use of an antagonist anti-VEGF antibody in the preparation of a medicament for treating multiple myeloma in a patient relapsed from or refractory to bortezomib therapy.

7. The use according to claim 6 wherein the anti-VEGF is for administration to the relapsed or refractory patient as a single agent.

8. The use according to claim 6 or claim 7, wherein the anti-VEGF antibody is selected from the group consisting of a Fab, Fv, F(ab')2, a scFV, a diabody and a bispecific antibody.

9. The use according to claim 6 or claim 7, wherein the anti-VEGF antibody is a human or humanized anti-VEGF antibody.

10. The use according to claim 6 or claim 7 wherein the anti-VEGF antibody is bevacizumab.

## Patentansprüche

1. Antagonistischer Anti-VEGF-Antikörper zur Verwendung in einem Verfahren zur Behandlung von multiplem Myelom bei einem Patienten, der nach Bortezomibtherapie rückfällig ist oder nicht darauf anspricht.

2. Anti-VEGF-Antikörper zur Verwendung in einem Verfahren zur Behandlung von multiplem Myelom nach Anspruch 1, worin dem rückfälligen oder nicht ansprechenden Patienten der Anti-VEGF-Antikörper als einzelnes Mittel verabreicht wird.

3. Anti-VEGF-Antikörper zur Verwendung in einem Verfahren zur Behandlung von multiplem Myelom nach Anspruch 1 oder Anspruch 2, worin der Anti-VEGF-Anti-körper aus der aus einem Fab, einem Fv, einem F(ab')₂, einem scFv, einem Diabody und einem bispezifischen Antikörper bestehenden Gruppe ausgewählt ist.

4. Anti-VEGF-Antikörper zur Verwendung in einem Verfahren zur Behandlung von multiplem Myelom nach Anspruch 1 oder Anspruch 2, worin der Anti-VEGF-Anti-körper ein menschlicher oder humanisierter Anti-VEGF-Antikörper ist.

5. Anti-VEGF-Antikörper zur Verwendung in einem Verfahren zur Behandlung von multiplem Myelom nach Anspruch 1 oder Anspruch 2, worin der Anti-VEGF-Anti-körper Bevacizumab ist.

6. Verwendung eines antagonistischen Anti-VEGF-Antikörpers zur Herstellung eines Medikaments zur Behandlung von multiplem Myelom bei einem Patienten, der nach Bortezomibtherapie rückfällig ist oder nicht darauf anspricht.

7. Verwendung nach Anspruch 6, worin der Anti-VEGF der Verabreichung an den rückfälligen oder nicht ansprechenden Patienten als einzelnes Mittel dient.

8. Verwendung nach Anspruch 6 oder Anspruch 7, worin der Anti-VEGF-Antikör-per aus der aus einem Fab, einem Fv, einem F(ab')₂, einem scFv, einem Diabody und einem bispezifischen Antikörper bestehenden Gruppe ausgewählt ist.

9. Verwendung nach Anspruch 6 oder Anspruch 7, worin der Anti-VEGF-Antikör-per ein menschlicher oder humanisierter Anti-VEGF-Antikörper ist.

10. Verwendung nach Anspruch 6 oder Anspruch 7, worin der Anti-VEGF-Antikör-per Bevacizumab ist.

## Revendications

1. Anticorps antagoniste anti-VEGF destiné à être utilisé dans un procédé de traitement du myélome multiple chez un patient ayant fait une rechute suite à une thérapie par bortézomib ou étant réfractaire à cette dernière.

2. Anticorps anti-VEGF destiné à être utilisé dans un procédé de traitement du myélome multiple selon la revendication 1, dans lequel l'anticorps anti-VEGF est administré en tant qu'agent unique au patient ayant fait une rechute ou étant réfractaire.

3. Anticorps anti-VEGF destiné à être utilisé dans un procédé de traitement du myélome multiple selon la revendication 1 ou la revendication 2, dans lequel l'anticorps anti-VEGF est choisi dans le groupe constitué des fragments Fab, Fv, F(ab')2, scFV, d'un fragment d'anticorps dimérique et d'un anticorps bispécifique.

4. Anticorps anti-VEGF destiné à être utilisé dans un procédé de traitement du myélome multiple selon la revendication 1 ou 2, dans lequel l'anticorps anti-VEGF est un anticorps anti-VEGF humain ou humanisé.

5. Anticorps anti-VEGF destiné à être utilisé dans un procédé de traitement du myélome multiple selon la revendication 1 ou 2, dans lequel l'anticorps anti-VEGF est le bévacizumab.

6. Utilisation d'un anticorps antagoniste anti-VEGF dans la préparation d'un médicament pour le traitement du myélome multiple chez un patient ayant fait une rechute suite à une thérapie par bortézomib ou étant réfractaire à cette dernière.

7. Utilisation selon la revendication 6, dans laquelle l'anti-VEGF est destiné à être administré au patient ayant fait une rechute ou étant réfractaire en tant qu'agent unique.

8. Utilisation selon la revendication 6 ou la revendication 7, dans laquelle l'anticorps anti-VEGF est choisi dans le groupe constitué des fragments Fab, Fv, F(ab')2, scFV, d'un fragment d'anticorps dimérique et d'un anticorps bispécifique.

9. Utilisation selon la revendication 6 ou la revendication 7, dans laquelle l'anticorps anti-VEGF est un anticorps anti-VEGF humain ou humanisé.

10. Utilisation selon la revendication 6 ou la revendication 7, dans laquelle l'anticorps anti-VEGF est le bévacizumab.
